# EUROPEAN PATENT APPLICATION

(11) **EP 4 667 486 A1**
(43) Date of publication of application: **24.12.2025**
(21) Application number: 24757148.2
(22) Date of filing: 08.02.2024
(51) Int. Cl.: C07K 14/705, A61P 37/00, A61K 38/00

(54) **COMPOSITION COMPRISING CD38 OR FRAGMENT THEREOF FOR TREATING AUTOIMMUNE DISEASES**

(30) Priority: 16.02.2023 KR 20230020897; 07.02.2024 KR 20240018964
(71) Applicant: DNT Co., Ltd., Jeonju-si Jeonbuk-do 54969 (KR)
(72) Inventor: KIM, Uh-Hyun, Jeonju-si Jeonbuk-do 54823 (KR); NAM, Tae-Sik, Iksan-si Jeonbuk-do 54647 (KR)
(74) Representative: V.O.
(86) International application number: PCT/KR2024/001940
(87) International publication number: WO 2024/172419

(57) **Abstract**

The present disclosure relates to a polypeptide including a fragment of CD38. In addition, the present disclosure relates to a composition containing CD38 or a fragment thereof for preventing or treating autoimmune diseases. The CD38 or the fragment thereof, according to the present disclosure, increases regulatory T-cells (Tregs) and alleviates various symptoms of autoimmune diseases, and thus can be used as an agent for preventing or treating autoimmune diseases.

## Description

### [Technical Field]

The present disclosure relates to a composition for preventing or treating autoimmune diseases, which contains soluble CD38 or a fragment thereof.

### [Background Art]

An autoimmune disease is a disease that occurs as an immune response to the body's own cells or tissues. It is a disease in which the immune system incorrectly responds to normal physiological substances and some of the body's cells. Autoimmune diseases include about 100 types of diseases, and representative examples include rheumatoid arthritis, autoimmune thyroiditis, psoriasis, inflammatory bowel diseases such as Crohn's disease, lupus (systemic lupus erythematosus), multiple sclerosis, type 1 diabetes (insulin-dependent), etc.

It is believed that the cause of autoimmune diseases rapid changes in the human living environment. A sharp decline in parasitic and bacterial infections is also believed to be one of causes. The body's immune system has evolved over a long period of time to resist various parasitic and bacterial infections. But, the development of modern antibiotics and anti-parasitic drugs has eliminated many sources of infection to which the immune system responds, causing the body's immune system to overreact to various environmental factors that it previously did not recognize as antigens. They include allergens that are harmless to the human body, such as pollen, house dust, and mites, as well as various autoantigens of the body.

In addition, increased stress due to modern complex social activities, hormonal abnormalities, and abnormal reactions of the body to environmental pollutants and various food additives are also pointed out as causes of autoimmune diseases.

Currently, adrenocortical hormones and small molecule drugs such as immunosuppressants, as well as antibody drugs, etc., are leading the global pharmaceutical market for the treatment of autoimmune diseases. Steroids, which are adrenocortical hormone drugs, are commonly used to treat autoimmune diseases, and other immunosuppressants are also used. However, both drugs are not suitable for long-term use because they lower the body's overall immunity. The adrenocortical hormone drugs include prednisone, dexamethasone, triamcinolone, betamethasone, fluticasone, etc. The immunosuppressants include cyclosporine, cyclophosphamide, azathioprine, chlorambucil, methotrexate, etc. TNF-α inhibitors, which have been developed relatively recently, have fewer side effects and are more selective and effective than general immunosuppressants. The TNF-α inhibitors currently on the market include etanercept, infliximab, etc. Since they are proteins as antibody drugs, they cannot be taken orally and should be injected using syringes. The use of TNF-α inhibitors may be limited because they are susceptibility to microbial infections during long-term treatment (Simard et al., Open J Rheumatol Autoimmune Dis. 14, 2012), may worsen multiple sclerosis, and may affect the homeostasis of oligodendrocytes, which are important for myelin formation in brain tissue (Kemanetzoglou & Andreadou. Curr Neurol Neurosci Rep. 17: 36, 2017, Napolitano et al., G Ital Dermatol Venereol. 153: 567, 2018).

Looking at the current status of development of therapeutic agents for autoimmune diseases, therapeutic agents inducing immune tolerance through regulation of co-stimulatory factors, therapeutic agents using regulation of regulatory T cells (Tregs) and T17 cells, and therapeutic agents based on dendritic cells are being developed. Inhibition of co-stimulatory factors induces T cell inactivation. Recently, anti-CD3 antibody therapy using antibodies against the T-cell receptor (TCR) has been attempted. Teplizumab, which is an anti-CD3 antibody, induces non-responsiveness in autoimmune T cells and increases tolerance. Teplizumab is undergoing clinical trials for autoimmune type 1 diabetes (Gaglia & Kissler. Biochemistry. 58: 4107, 2019). Especially, in patients with early-stage diabetes, teplizumab has been reported to promote not only T cell tolerance but also Treg proliferation (Christen & Kimmel. Front Endocrinol. 11: 591083, 2020). In addition, therapeutic agents such as abatacept and belatacept, which are CTLA4-Ig fusion proteins, were developed as T-cell surface proteins that induce binding to the costimulatory factor B7 utilizing a protein structure similar to the structure of CD28 (Paul et al., Clin Exp Rheumatol. 38: 1008, 2020). CTLA4-Ig acts as a competitive inhibitor with CD28 to inhibit co-stimulatory signals and induce tolerance in T cells by binding to B7 on antigen-presenting cells (Ouewole-said et al., Front Immunol. 11: 608024, 2020). These drugs are used to treat rheumatoid arthritis. LFA3-Ig (alefacept), which inhibits CD3-LFA3, was approved by the FDA for the treatment of psoriasis. It is being used as an autoimmune suppressant for renal transplantation, and is under clinical trial for type 1 diabetes (Georgakopoulou et al., Acta Dermatovenerol Croat. 21: 24, 2013).

The activation and regulatory mechanisms of Treg/Th17 cells in autoimmune regulation may be important therapeutic technologies for autoimmune diseases. Treg activity acts as an inhibitory factor which maintains immune tolerance and prevents autoimmune responses, and Th17 cells regulate autoimmune and inflammatory responses (Amaya-Uribe et al., J Autoimmun. 99: 52. 2019). The expression of IL-17 released from these cells is reported to be a major cause of autoimmune diseases. Therapeutic techniques using IL-17 monoclonal antibodies, etc. to suppress IL-17 are being utilized in lupus research, etc. (Robert & Miossec. Lupus. 29: 6, 2020). In addition, researches are underway to block signals such as IL-1, IL-23 and IL-6 to suppress the activation mechanism of Th17 cells, and studies on inhibitory signal cytokines such as IL-27 are also underway (Tran et al., Acta Trop. 6: 105-823, 2021). Researches are currently underway focusing on the FoxP3 gene, which is known to play an important role in the differentiation process of Treg cells. The decrease in the expression of FOXP3 is suggested to be an important cause in the autoimmune response process (Ali et al., Cells. 11; 9(12): 2665, 2020). Tregs secrete inhibitory cytokines such as IL-10 and TGF-β, and have functions related to suppression of Th12 activity such as absorption of IL-2, etc. (Terry & Oo. Front Immunol. 11: 565518, 2020). Tregs have been reported to signal through the signaling pathways of costimulatory factors such as CTLA-4 and LFA-1 (Tocci et al., Expert Rev Clin Immunol. 10: 1395, 2014). Therefore, the mechanism of Treg activation and regulation can be utilized as a major mechanism for controlling Th17-related autoimmune diseases and can be the subject for development of a therapeutic agent (Ohue & Nishikawa. Cancer Sci. 110: 2080, 2019).

T cell tolerance can be induced by inhibiting the maturation of dendritic cells, which are antigen-presenting cells (De Winde et al., Med Microbiol Immunol. 209: 515, 2020). Additionally, a cell therapy agent which induces suppressive immune tolerance by inducing undifferentiated dendritic cells into tolerogenic cells for the purpose of activating T cells is being developed (Philips et al., Front Immunol 8: 1279).

CD38 was shown to be a type II transmembrane protein through molecular cloning [Jackson & Bell. J Immunol. 144: 2811, 1990]. CD38 was found to be a member of the NADase family with sequence homology to Aplysia ADP-ribosyl cyclase (ARC) through cloning (States et al., Trends Biochem. 17: 495, 1992). Enzymological studies have revealed that CD38 is an enzyme that generates the Ca²⁺ signaling messengers cADPR and NAADP (Howard et al., Science. 262: 1056, 1993; Aarhus et al., J Biol Chem. 270: 30327, 1995).

The matters described above as background art are intended only to enhance understanding of the background of the present disclosure, and should not be taken as an acknowledgment that they correspond to prior art already known to those skilled in the art.

### [Disclosure]

### [Technical Problem]

The inventors of the present disclosure have confirmed that soluble CD38 known in mammals or a fragment thereof may be used to treat autoimmune diseases by effectively inducing immune tolerance, and have completed the present disclosure.

Therefore, the present disclosure is directed to providing a polypeptide including an amino acid sequence represented by the following formula (from N-terminus to C-terminus):

X1-L-Q-C-V-K-N-P-E-X2-X3-S-C (formula I),

wherein
X1 is F or L;
X2 is H or D; and
X3 is P or S.

The present disclosure is also directed to providing a nucleic acid molecule encoding the polypeptide.

The present disclosure is also directed to providing a vector including the nucleic acid molecule.

The present disclosure is also directed to providing a host cell transformed with the vector.

The present disclosure is also directed to providing a pharmaceutical composition for preventing or treating a disease or a disorder, which contains the polypeptide, a nucleic acid molecule encoding the polypeptide, a vector including the nucleic acid molecule, or an isolated cell including the vector, and a pharmaceutically acceptable carrier.

The present disclosure is also directed to providing a pharmaceutical composition for preventing or treating an autoimmune disease, which contains the polypeptide, a nucleic acid molecule encoding the polypeptide, a vector including the nucleic acid molecule, or an isolated cell including the vector, and a pharmaceutically acceptable carrier.

The present disclosure is also directed to providing a pharmaceutical composition for preventing or treating rheumatoid arthritis, which contains the polypeptide, a nucleic acid molecule encoding the polypeptide, a vector including the nucleic acid molecule, or an isolated cell including the vector, and a pharmaceutically acceptable carrier.

The present disclosure is also directed to providing a pharmaceutical composition for preventing or treating uveitis, which contains the polypeptide, a nucleic acid molecule encoding the polypeptide, a vector including the nucleic acid molecule, or an isolated cell including the vector, and a pharmaceutically acceptable carrier.

The present disclosure is also directed to providing a pharmaceutical composition for preventing or treating inflammatory bowel disease, which contains the polypeptide, a nucleic acid molecule encoding the polypeptide, a vector including the nucleic acid molecule, or an isolated cell including the vector, and a pharmaceutically acceptable carrier.

The present disclosure is also directed to providing a method for preventing or treating a disease or a disorder, which includes a step of administering the polypeptide, a nucleic acid molecule encoding the polypeptide, a vector including the nucleic acid molecule, or an isolated cell including the vector to a subject in need thereof.

The present disclosure is also directed to providing a method for preventing or treating an autoimmune disease, which includes a step of administering the polypeptide, a nucleic acid molecule encoding the polypeptide, a vector including the nucleic acid molecule, or an isolated cell including the vector to a subject in need thereof.

The present disclosure is also directed to providing a method for preventing or treating inflammatory bowel disease, which includes a step of administering the polypeptide, a nucleic acid molecule encoding the polypeptide, a vector including the nucleic acid molecule, or an isolated cell including the vector to a subject in need thereof.

Other objects and advantages of the present disclosure will become more apparent from the detailed description of the invention, the claims and the drawings that follow.

### [Technical Solution]

For the sake of ease of understanding of the disclosure in this specification, a number of terms and phrases will be defined. Additional definitions are provided throughout the detailed description.

### I. Definition

Throughout the present disclosure, the term "a" or "an" is understood to refer to one or more of the corresponding entity. For example, "a polypeptide" is understood to refer to one or more polypeptides. Likewise, the terms "a" (or "an"), "one or more" and "at least one" may be used interchangeably herein.

When aspects are described herein as "comprising," it may be understood that other similar aspects described as "consisting of" and/or "consisting essentially of" are also provided.

Unless defined otherwise, all technical and scientific terms used herein have the same meanings as commonly understood by one of ordinary skill in the art to which the present disclosure relates. For example, Concise Dictionary of Biomedicine and Molecular Biology, Juo, Pei-Show, 2nd ed., 2002, CRC Press; Dictionary of Cell and Molecular Biology, 3rd ed, 1999, Academic Press; and Oxford Dictionary Of Biochemistry and Molecular Biology, Revised, 2000, Oxford University Press provide one of ordinary skill in the art with general dictionaries of many of the terms used in the present disclosure.

Units, prefixes and symbols are indicated in the form accepted by the Systeme International de Unites (SI). A numeric range includes the numbers that define that range. Unless indicated otherwise, amino acid sequences are written from left to right in amino-to-carboxyl direction. The headings provided herein are not intended to limit the various aspects of the disclosure and are determined in consideration of the specification as a whole. Accordingly, the terms defined below are more fully defined by referring to the specification as a whole.

As used herein, the terms "administration," "administering," and grammatical variations thereof refer to introducing a composition (e.g., a composition containing the polypeptide, nucleic acid molecule, vector, or isolated cell described herein) into a subject via a pharmaceutically acceptable route. The composition is introduced to the subject by any suitable route, including oral, cutaneous, intrapulmonary, intranasal, parenteral (intravenous, intraarterial, intramuscular, intraperitoneal, or subcutaneous), rectal, intralymphatic, intrathecal, periocular, intraocular, or topical administration. The administration includes self-administration and administration by others. The composition or preparation exerts an intended function through a suitable route of administration. For example, if the suitable route is intravenous, the composition or formulation is administered by introducing into the vein of a subject.

The term "conserved" as used herein refers to polypeptide sequences or nucleotides or amino acid residues of a polynucleotide sequence, which appear unchanged at the same position in two or more sequences being compared. Relatively conserved nucleotides or amino acids are those that are more conserved in a sequence than the nucleotides or amino acids that occur at different positions in the sequence.

The term "amino acid" as used herein includes the 22 standard amino acids (arginine (R), lysine (K), histidine (H), glutamic acid (E), aspartic acid (D), glutamine (Q), asparagine (N), leucine (L), isoleucine (I), valine (V), methionine (M), phenylalanine (F), tryptophan (W), tyrosine (Y), glycine (G), alanine (A), serine (S), threonine (T), proline (P), cysteine (C), etc.) which are naturally incorporated into peptides, as well as D-isomers and modified amino acids. Additionally, the peptide may contain non-standard amino acids that have been modified post-translationally. The post-translational modification may include phosphorylation, glycosylation, acylation (e.g., acetylation, myristoylation and palmitoylation), alkylation, carboxylation, hydroxylation, glycation, biotinylation, ubiquitinylation, changes in chemical properties (e.g., beta-elimination deimidation and deamidation) and structural changes (e.g., formation of disulfide bridges), although not being limited thereto. The peptide may be a wild-type peptide identified and isolated from a natural source. Meanwhile, the peptide may be an artificial variant including an amino acid sequence in which one or more amino acids are substituted, deleted and/or inserted. Changes in amino acids in a wild-type polypeptide as well as in an artificial variant include conservative amino acid substitution that does not significantly affect the folding and/or activity of a protein. For example, the conservative substitution may include substitution with basic amino acids (arginine (R), lysine (K), and histidine (H)), acidic amino acids (glutamic acid (E) and aspartic acid (D)), polar amino acids (glutamine (Q) and asparagine (N)), hydrophobic amino acids (leucine (L), isoleucine (I), valine (V), and methionine (M)), aromatic amino acids (phenylalanine (F), tryptophan (W), and tyrosine (Y)), and small amino acids (glycine (G), alanine (A), serine (S), and threonine (T)). Amino acid substitutions that generally do not alter specific activity are known in the art. The most common exchanges may include Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly.

In some aspects, two or more sequences are said to be "completely conserved" or "identical" if they are 100% identical to each other. In some aspects, two or more sequences are said to be "highly conserved" if they are at least 70% identical, at least 80% identical, at least 90% identical, or at least 95% identical to each other. In some aspects, two or more sequences are said to be "highly conserved" if they are about 70% identical, about 80% identical, about 90% identical, about 95% identical, about 98% identical, or about 99% identical to each other. In some aspects, two or more sequences are said to be "conserved" if they are at least 30% identical, at least 40% identical, at least 50% identical, at least 60% identical, at least 70% identical, at least 80% identical, or at least 90% identical, or at least 95% identical to each other. In some aspects, two or more sequences are said to be "conserved" if they are about 30% identical, about 40% identical, about 50% identical, about 60% identical, about 70% identical, about 80% identical, about 90% identical, about 95% identical, about 98% identical, or about 99% identical to each other. Conservation of sequences may be applied to the entire length of a polynucleotide or a polypeptide, or to a part, region or feature thereof.

The terms "complementary" and "complementarity" refer to two or more oligomers (i.e., each including a nucleobase sequence), or an oligomer and a target gene that are associated with each other by the Watson-Crick base pair rules. For example, the nucleobase sequence "T-G-A (5' → 3') is complementary to the nucleobase sequence "A-C-T (3' → 5').

The complementarity may be "partial" if fewer than all the nucleobases of a given nucleobase sequence match another nucleobase sequence, according to the base pair rules. For example, in some aspects, the complementarity between a given nucleobase sequence and another nucleobase sequence may be about 70%, about 75%, about 80%, about 85%, about 90%, or about 95%. Thus, in certain aspects, the term "complementary" refers to an identity or complementarity to a target nucleic acid sequence of at least about 80%, at least about 85%, at least about 90%, at least about 91%, at least about 92%, at least about 93%, at least about 94%, at least about 95%, at least about 96%, at least about 97%, at least about 98%, or at least about 99%. Or, there may be "complete" or "perfect" (100%) complementarity between a given nucleobase sequence and another nucleobase sequence. In some aspects, the degree of complementarity between nucleotide sequences significantly affects the efficiency and strength of hybridization between the sequences.

The term "expression" as used herein refers to a process by which a polynucleotide produces a gene product, such as an RNA or a polypeptide. This includes, but is not limited to, transcribing a polynucleotide into a messenger RNA (mRNA) and translating the mRNA into a polypeptide. The expression produces a "gene product". The gene product used herein may be a nucleic acid, such as an RNA, produced by transcription of a gene. The gene product used herein may be a nucleic acid or a polypeptide translated from a transcriptome. The gene product described herein further includes a nucleic acid modified after transcription, e.g., by polyadenylation or splicing, or a polypeptide modified after translation, e.g., by phosphorylation, methylation, glycosylation, lipidation, association with another protein subunit, or proteolytic cleavage.

The term "identity" as used herein refers to conservation of overall monomers between polymer molecules, e.g., polynucleotide molecules. The term "identical" without any additional modifier, e.g., a polynucleotide A being identical to a polynucleotide B, means that the polynucleotide sequences are 100% identical (100% sequence identity). The description that two sequences are, e.g., "70% identical" is equivalent to describing that they have, e.g., "70% sequence identity."

For example, the calculation of the (percent) identity of two polypeptide or polynucleotide sequences can be performed by aligning the two sequences for optimal comparison purposes (e.g., gaps can be introduced into one or both of the first and second polypeptide or polynucleotide sequences for optimal alignment, and unidentical sequences can be ignored for comparison purposes). In certain aspects, the length of the aligned sequences for comparison is at least 30%, at least 40%, at least 50%, at least 60%, at least 70%, at least 80%, at least 90%, at least 95% or 100% of the length of the reference sequence. Afterwards, the amino acids at the corresponding amino acid positions or, nucleotides in the case of polynucleotides, are compared.

If a particular position in the first sequence is occupied by an amino acid or nucleotide that is identical to a corresponding position in the second sequence, the molecules are identical at that position. The (percent) identity between two sequences is a function of the number of identical positions shared by the sequences, in consideration of the number of gaps that need to be introduced for optimal alignment of the two sequences and the length of each of the gaps. Sequence comparison and determination of (percent) identity between two sequences can be accomplished using mathematical algorithms.

Suitable software programs that can be used to align different sequences (e.g., polynucleotide sequences) are available from a number of sources. One suitable program for determining the sequence (percent) identity is Bl2seq, which is a part of the BLAST program suite available from the U.S. government's National Center for Biotechnology Information BLAST website (blast.ncbi.nlm.nih.gov). Bl2seq performs comparisons between two sequences using the BLASTN or BLASTP algorithm. BLASTN is used to compare nucleic acid sequences, whereas BLASTP is used to compare amino acid sequences. Other suitable programs include Needle, Stretcher, Water or Matcher, which are parts of the bioinformatics program EMBOSS suite and are also available from the European Bioinformatics Institute (EBI) at www.ebi.ac.uk/Tools/psa.

Sequence arrangement may be performed using methods known in the art such as MAFFT, Clustal (ClustalW, Clustal X or Clustal Omega), MUSCLE, etc.

Different regions within a single polynucleotide or polypeptide target sequence that are aligned with a polynucleotide or polypeptide reference sequence can each have their own sequence (percent) identity. The sequence identity (percent) values are rounded to the nearest decimal point. For example, 80.11, 80.12, 80.13 and 80.14 are rounded to 80.1, and 80.15, 80.16, 80.17, 80.18 and 80.19 are rounded to 80.2. And, the length values are always integer values.

In certain aspects, the percent identity (%ID) of a first amino acid sequence (or nucleic acid sequence) to a second amino acid sequence (or nucleic acid sequence) is calculated as %ID = 100 x (Y/Z), where Y is the number of amino acid residues (or nucleobases) that score as identical matches when the first and second sequences are aligned (by visual inspection or by a particular sequence alignment program), and Z is the total number of residues in the second sequence. If the length of a first sequence is longer than that of a second sequence, the (percent) identity of the first sequence to the second sequence will be higher than the (percent) identity of the second sequence to the first sequence.

Those skilled in the art will appreciate that the generation of sequence alignments for calculating sequence (percent) identity is not limited to binary sequence-sequence comparisons treated entirely by primary sequence data. It will also be appreciated that sequence alignments may be achieved by integrating sequence data with data from heterogeneous sources, such as structural data (e.g., crystallographic protein structure), functional data (e.g., positions of mutations), or phylogenetic data. A suitable program for integrating heterogeneous data into multiple sequence alignments is available at www.tcoffee.org, or, alternatively, T-Coffee available from EBI may also be used. It will also be appreciated that the final alignment used to calculate the sequence (percent) identity may be systematized automatically or manually.

The term "fragment" as used herein refers to a fragment (e.g., sequences of SEQ ID NOS: 1 to 30) derived from a full-length sequence (e.g., full-length sCD38). The fragment is meant to exclude the full-length sequence.

In some aspects, the nucleic acid molecule or polynucleotide described herein may include a promoter and/or other expression (e.g., transcription) regulatory elements operably linked to one or more coding regions. In the operable linkage, a coding region for a gene product is linked to one or more regulatory regions in such a way that expression of the gene product is under the influence or control of the regulatory region(s). For example, a coding region and a promoter are "operably linked" if the induction of promoter function causes transcription of mRNA which encodes a gene product encoded by the coding region and the nature of the linkage between the promoter and the coding region does not interfere with the ability of the promoter to direct expression of the gene product or the ability of the DNA template to be transcribed. Expression control elements other than promoter, such as an enhancer, an operator, a repressor and a transcription termination signal, may also be operably linked to a coding region which directs the expression of a gene product.

The terms "subject," "patient," "individual" and "host", and variations thereof, as used herein, are interchangeable and refer to any mammalian subject to which any of the compositions described herein (e.g., a polypeptide, a polynucleotide, a vector, a cell, or a pharmaceutical composition) is administered. Non-limiting examples include human, domestic animals (e.g., dog, cat, etc.), farm animals (e.g., cow, sheep, pig, horse, etc.), and laboratory animals (e.g., monkey, rat, mouse, rabbit, guinea pig, etc.), particularly human, in need of diagnosis, treatment or therapy. The methods described herein are applicable to prophylactic or therapeutic uses for both human and animals.

The phrase "subject in need" as used herein includes a subject, such as a mammalian subject, that would benefit from the administration of the compositions described herein.

The term "therapeutically effective amount" as used herein refers to an amount of a reagent or a pharmaceutical compound including the composition of the present disclosure sufficient to produce a desired therapeutic, pharmacological and/or physiological effect in a subject in need thereof. The therapeutically effective amount may be a "prophylactically effective amount" because prophylaxis can be seen as treatment.

The terms "treat," "treatment" or "treating", as used herein, refer to, for example, reducing the severity of a disease or a disorder, reducing the duration of a disease, improving or eliminating one or more symptoms associated with a disease or a disorder, or providing a beneficial effect to a subject suffering from a disease or a disorder without necessarily curing the disease or disorder. The term also includes preventing or inhibiting a disease or a disorder or symptoms thereof.

The term "vector" or "construct" as used herein refers to any vehicle into which a nucleic acid molecule or a gene can be inserted, e.g., a delivery vehicle into which a nucleic acid sequence can be inserted, and can be introduced into a cell where it can be replicated. The nucleic acid sequence that can be inserted into the vector may be exogenous or heterologous. The nucleic acid sequence may be full-length sCD38 or a fragment thereof. Examples of the construct include, but are not limited to, a plasmid, a cosmid, and a virus (e.g., AAV). Those skilled in the art can produce such vectors or constructs by standard recombinant techniques (see, e.g., Maniatis, et al., Molecular Cloning, A Laboratory Manual, Cold Spring Harbor Press, Cold Spring Harbor, N.Y., 1988; and Ausubel et al., In: Current Protocols in Molecular Biology, John, Wiley & Sons, Inc, NY, 1994). The term "expression vector" or "expression construct", as used herein, means a vector or a construct including a nucleotide sequence that encodes at least a portion of a gene product to be transcribed. In some cases, an RNA molecule is then translated into a protein, a polypeptide, or a peptide. The expression construct may include a variety of regulatory elements. In addition to regulatory sequences that control transcription and translation, the vector and the expression vector may also include nucleotide sequences that provide other functions. Viruses that can be used in the present disclosure include, but are not limited to, retrovirus, herpes simplex virus, lentivirus, poxvirus, vaccinia virus, rhabdovirus, adenovirus, helper-dependent adenovirus, adeno-associated virus (AAV), etc.

The vector may be engineered to encode a selectable marker or a reporter that is provided for selection or identification of a cell into which the vector is incorporated. The expression of a selectable marker or a reporter allows identification and/or selection of a host cell in which different coding regions included in the vector are integrated and expressed. Examples of selectable marker genes known and used in the art include genes providing resistance to ampicillin, streptomycin, gentamicin, kanamycin, hygromycin, bialaphos herbicides, sulfonamides, etc.; and genes used as phenotypic markers, such as anthocyanin regulatory genes, isopentanyl transferase genes, etc. Examples of reporters known and used in the art include luciferase (Luc), green fluorescent protein (GFP), chloramphenicol acetyltransferase (CAT), β-galactosidase (LacZ), β-glucuronidase (Gus), etc. The selectable marker can also be seen as a reporter.

The term "cell" as used herein includes eukaryotic and prokaryotic cells and means any transformable cell capable of replicating the vector or expressing a gene encoded by the vector. A cell can be transfected, transduced or transformed by the vector, which refers to a process by which an exogenous polynucleotide (nucleic acid molecule) is delivered or introduced into a host cell. In this specification, the term "transformation" is used to include transfection and transduction.

The (host) cell of the present disclosure is not limited. But, it may be specifically an insect cell or a mammalian cell, more specifically Sf9 as an insect cell, or HEK293 cells, HeLa cells, ARPE-19 cells, RPE-1 cells, HepG2 cells, Hep3B cells, Huh-7 cells, C8D1a cells, Neuro2A cells, CHO cells, MES13 cells, BHK-21 cells, COS7 cells, COP5 cells, A549 cells, MCF-7 cells, HC70 cells, HCC1428 cells, BT-549 cells, PC3 cells, LNCaP cells, Capan-1 cells, Panc-1 cells, MIA PaCa-2 cells, SW480 cells, HCT166 cells, LoVo cells, A172 cells, MKN-45 cells, MKN-74 cells, Kato-III cells, NCI-N87 cells, HT-144 cells, SK-MEL-2 cells, SH-SY5Y cells, C6 cells, HT-22 cells, PC-12 cells, NIH3T3 cells, etc. as mammalian cells. In some aspects, the host cell is an isolated host cell.

### II. Polypeptide

The present disclosure relates to a polypeptide including an amino acid sequence of CD38 (e.g., soluble CD38) or a fragment (functional fragment) thereof. The polypeptide has an activity of reducing TNF-α level. The polypeptide has an activity of reducing IFN-γ. The present polypeptide has an activity of reducing IL-17 level. This polypeptide has an activity of increasing the amount of Treg cells.

Specifically, the present disclosure provides a polypeptide including an amino acid sequence represented by the following formula (from N-terminus to C-terminus):

X1-L-Q-C-V-K-N-P-E-X2-X3-S-C (formula I),

wherein
X1 is F or L;
X2 is H or D; and
X3 is P or S.

According to a specific exemplary embodiment of the present disclosure, the polypeptide includes an amino acid sequence selected from a group consisting of SEQ ID NOS: 1 to 3.

The sequence of SEQ ID NO: 1 is FLQCVKNPEHPSC, which is the sCD38 fragment sequence of mouse, rat and horse. The sequence of SEQ ID NO: 2 is FLQCVKNPEDSSC, which is the sCD38 fragment sequence of human and chimpanzee. The sequence of SEQ ID NO: 3 is LLQCVKNPEHSSC and is the sequence of the sCD38 fragment of dog.

According to an exemplary embodiment of the present disclosure, the sCD38 fragment consisting of 13 amino acids (e.g., H13 (284-296)) reduces TNF-α secretion from human dendritic cells induced by LPS (Example 11).

According to a specific exemplary embodiment of the present disclosure, the polypeptide may be a polypeptide including an amino acid sequence represented by the following formula (from N-terminus to C-terminus):

X4-X1-L-Q-C-V-K-N-P-E-X2-X3-S-C (formula II),

wherein
X4 is R or K;
X1 is F or L;
X2 is H or D; and
X3 is P or S.

In some aspects, the polypeptide includes one or more amino acid sequence selected from a group consisting of SEQ ID NOS: 4 to 6.

The sequence of SEQ ID NO: 4 is RFLQCVKNPEHPSC, which is the sCD38 fragment sequence of mouse, rat and horse. The sequence of SEQ ID NO: 5 is KFLQCVKNPEDSSC, which is the sCD38 fragment sequence of human and chimpanzee. The sequence of SEQ ID NO: 6 is RLLQCVKNPEHSSC, which is the sequence of the sCD38 fragment of dog.

According to an exemplary embodiment of the present disclosure, the sCD38 fragment consisting of 14 amino acids (e.g., M14 (287-300)) reduces TNF-α secretion from human dendritic cells induced by LPS (Example 4).

According to a specific exemplary embodiment of the present disclosure, the polypeptide includes an amino acid sequence selected from a group consisting of SEQ ID NOS: 7 to 10.

The sequence of SEQ ID NO: 7 is RFLQCVKNPEHPSCR, which is the sCD38 fragment sequence of mouse and rat. The sequence of SEQ ID NO: 8 is KFLQCVKNPEDSSCT, which is the sCD38 fragment sequence of human and chimpanzee. The sequence of SEQ ID NO: 9 is RLLQCVKNPEHSSCK, which is the sCD38 fragment sequence of dog. The sequence of SEQ ID NO: 10 is RFLQCVKNPEHPSCT, which is the sCD38 fragment sequence of horse.

According to a specific exemplary embodiment of the present disclosure, the polypeptide includes an amino acid sequence selected from a group consisting of SEQ ID NOS: 11 to 15.

The sequence of SEQ ID NO: 11 is RFLQCVKNPEHPSCRL, which is the sCD38 fragment sequence of mouse. The sequence of SEQ ID NO: 12 is RFLQCVKNPEHPSCRL, which is the sCD38 fragment sequence of rat. The sequence of SEQ ID NO: 13 is KFLQCVKNPEDSSCTS, which is the sCD38 fragment sequence of human and chimpanzee. The sequence of SEQ ID NO: 14 is RLLQCVKNPEHSSCKY, which is the sCD38 fragment sequence of dog. The sequence of SEQ ID NO: 15 is RFLQCVKNPEHPSCTS, which is the sCD38 fragment sequence of horse.

According to an exemplary embodiment of the present disclosure, the sCD38 fragment consisting of 16 amino acids (e.g., M16 (287-302)) reduces TNF-α secretion from human dendritic cells induced by LPS (Example 4).

According to a specific exemplary embodiment of the present disclosure, the polypeptide includes an amino acid sequence selected from a group consisting of SEQ ID NOS: 16 to 20.

The sequence of SEQ ID NO: 16 is RFLQCVKNPEHPSCRLNT, which is the sCD38 fragment sequence of mouse. The sequence of SEQ ID NO: 17 is RFLQCVKNPEHPSCRLNV, which is the sCD38 fragment sequence of rat. The sequence of SEQ ID NO: 18 is KFLQCVKNPEDSSCTSEI, which is the sCD38 fragment sequence of human and chimpanzee. The sequence of SEQ ID NO: 19 is RLLQCVKNPEHSSCKYNL, which is the sCD38 fragment sequence of dog. The sequence of SEQ ID NO: 20 is RFLQCVKNPEHPSCTSGI, which is the sCD38 fragment sequence of horse.

According to an exemplary embodiment of the present disclosure, the sCD38 fragment consisting of 18 amino acids (e.g., H18 (283-300)) reduces TNF-α secretion from human dendritic cells induced by LPS (Example 11).

According to a specific exemplary embodiment of the present disclosure, the polypeptide includes an amino acid sequence selected from a group consisting of SEQ ID NOS: 21 to 25.

The sequence of SEQ ID NO: 21 is ARFLQCVKNPEHPSCRLNT, which is the sCD38 fragment sequence of mouse. The sequence of SEQ ID NO: 22 is VRFLQCVKNPEHPSCRLNV, which is the sCD38 fragment sequence of rat. The sequence of SEQ ID NO: 23 is DKFLQCVKNPEDSSCTSEI, which is the sCD38 fragment sequence of human and chimpanzee. The sequence of SEQ ID NO: 24 is VRLLQCVKNPEHSSCKYNL, which is the sCD38 fragment sequence of dog. The sequence of SEQ ID NO: 25 is VRFLQCVKNPEHPSCTSGI, which is the sCD38 fragment sequence of horse.

According to an exemplary embodiment of the present disclosure, the sCD38 fragment consisting of 19 amino acids (e.g., M19 (286-304)) prevents shortening of the colon due to ulcerative colitis and reduces the progression of ulcerative colitis (Example 9).

According to a specific exemplary embodiment of the present disclosure, the polypeptide includes an amino acid sequence selected from a group consisting of SEQ ID NOS: 26 to 30.

The sequence of SEQ ID NO: 26 is LKMIVQKRNMIFACVDNYRPARFLQCVKNPEHPSCRLNT, which is the sCD38 fragment sequence of mouse. The sequence of SEQ ID NO: 27 is LKSIVNKRNMIFACQDNYRPVRFLQCVKNPEHPSCRLNV, which is the sCD38 fragment sequence of rat. The sequence of SEQ ID NO: 28 is LESIISKRNIQFSCKNIYRPDKFLQCVKNPEDSSCTSEI, which is the sCD38 fragment sequence of human and chimpanzee. The sequence of SEQ ID NO: 29 is LKQILKTRNIIFICQNNYRPVRLLQCVKNPEHSSCKYNL, which is the sCD38 fragment sequence of dog. The sequence of SEQ ID NO: 30 is LKSIISERNITFTCQNNYRPVRFLQCVKNPEHPSCTSGI, which is the sCD38 fragment sequence of horse.

According to an exemplary embodiment of the present disclosure, the sCD38 fragment consisting of 39 amino acids (e.g., peptide 4: 266-304) reduces the progression of autoimmune uveitis (Example 5) and reduces rheumatoid arthritis (Example 8).

According to a specific exemplary embodiment of the present disclosure, the polypeptide may be a sequence of full-length CD38 (e.g., SEQ ID NOS: 31 to 36) or a fragment sequence thereof.

In some aspects, the polypeptide may consist of 13 to 300, 13 to 250, 13 to 240, 13 to 230, 13 to 220, 13 to 210, 13 to 200, 13 to 190, 13 to 180, 13 to 170, 13 to 160, 13 to 150, 13 to 140, 13 to 130, 13 to 120, 13 to 110, 13 to 100, 13 to 90, 13 to 80, 13 to 70, 13 to 60, or 13 to 50 amino acids.

In some aspects, the polypeptide may consist of 13 to 49, 13 to 48, 13 to 47, 13 to 46, 13 to 45, 13 to 44, 13 to 43, 13 to 42, 13 to 41, 13 to 40, or 13 to 39 amino acids.

According to a specific exemplary embodiment of the present disclosure, the CD38 may be soluble CD38.

The origin of the CD38 is not limited. But, it may be a CD38 derived specifically from a mammal, more specifically from dog, horse, mouse, rat, human or chimpanzee, most specifically from human.

In some aspects, the polypeptide has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, or at least 95% sequence identity to the amino acid sequences set forth in SEQ ID NOS: 1 to 36.

### III. Nucleic acid molecule

The present disclosure relates to a nucleic acid molecule which encodes a polypeptide, which includes an amino acid sequence of CD38 (e.g., soluble CD38) or a fragment (functional fragment) thereof. Specifically, the present disclosure provides a nucleic acid molecule which encodes a polypeptide including an amino acid sequence represented by the following formula (from N-terminus to C-terminus):

X1-L-Q-C-V-K-N-P-E-X2-X3-S-C (formula I),

wherein
X1 is F or L;
X2 is H or D; and
X3 is P or S.

According to a specific exemplary embodiment of the present disclosure, the nucleic acid molecule encodes a polypeptide including an amino acid sequence selected from a group consisting of SEQ ID NOS: 1 to 3.

According to a specific exemplary embodiment of the present disclosure, the nucleic acid molecule can encode a polypeptide including an amino acid sequence represented by the following formula (from N-terminus to C-terminus):

X4-X1-L-Q-C-V-K-N-P-E-X2-X3-S-C (formula II),

wherein
X4 is R or K;
X1 is F or L;
X2 is H or D; and
X3 is P or S.

According to a specific exemplary embodiment of the present disclosure, the nucleic acid molecule encodes a polypeptide including an amino acid sequence selected from a group consisting of SEQ ID NOS: 4 to 6.

According to a specific exemplary embodiment of the present disclosure, the nucleic acid molecule encodes a polypeptide including an amino acid sequence selected from a group consisting of SEQ ID NOS: 7 to 10.

According to a specific exemplary embodiment of the present disclosure, the nucleic acid molecule encodes a polypeptide including an amino acid sequence selected from a group consisting of SEQ ID NOS: 11 to 15.

According to a specific exemplary embodiment of the present disclosure, the nucleic acid molecule encodes a polypeptide including an amino acid sequence selected from a group consisting of SEQ ID NOS: 16 to 20.

According to a specific exemplary embodiment of the present disclosure, the nucleic acid molecule encodes a polypeptide including an amino acid sequence selected from a group consisting of SEQ ID NOS: 21 to 25.

According to a specific exemplary embodiment of the present disclosure, the nucleic acid molecule encodes a polypeptide including an amino acid sequence selected from a group consisting of SEQ ID NOS: 26 to 30.

According to a specific exemplary embodiment of the present disclosure, the nucleic acid molecule can encode a sequence of full-length CD38 (e.g., SEQ ID NOS: 31 to 36) or a fragment sequence thereof.

In some aspects, the nucleic acid molecule can encode a polypeptide consisting of 13 to 300, 13 to 250, 13 to 240, 13 to 230, 13 to 220, 13 to 210, 13 to 200, 13 to 190, 13 to 180, 13 to 170, 13 to 160, 13 to 150, 13 to 140, 13 to 130, 13 to 120, 13 to 110, 13 to 100, 13 to 90, 13 to 80, 13 to 70, 13 to 60, or 13 to 50 amino acids.

In some aspects, the nucleic acid molecule can encode a polypeptide consisting of 13 to 49, 13 to 48, 13 to 47, 13 to 46, 13 to 45, 13 to 44, 13 to 43, 13 to 42, 13 to 41, 13 to 40, or 13 to 39 amino acids.

According to a specific exemplary embodiment of the present disclosure, the CD38 may be soluble CD38.

The origin of the CD38 is not limited. But, it may be a CD38 derived specifically from a mammal, more specifically from dog, horse, mouse, rat, human or chimpanzee, most specifically from human.

In some aspects, the nucleic acid molecule has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, or at least 95% sequence identity to a sequence encoding a polypeptide including amino acid sequences set forth in SEQ ID NOS: 1 to 36.

### IV. Vector and cell

The present disclosure relates to a vector including a nucleic acid molecule which encodes a polypeptide including an amino acid sequence of CD38 (e.g., soluble CD38) or a fragment (functional fragment) thereof, or a cell transformed with or including the vector. Specifically, the present disclosure provides a vector including a nucleic acid molecule which encodes a polypeptide including an amino acid sequence represented by the following formula (from N-terminus to C-terminus), or a cell transformed with the vector:

X1-L-Q-C-V-K-N-P-E-X2-X3-S-C (formula I),

wherein
X1 is F or L;
X2 is H or D; and
X3 is P or S.

According to a specific exemplary embodiment of the present disclosure, the nucleic acid molecule is a vector including a nucleic acid molecule which encodes a polypeptide including an amino acid sequence selected from a group consisting of SEQ ID NOS: 1 to 3, or a cell transformed with the vector.

According to a specific exemplary embodiment of the present disclosure, the nucleic acid molecule may be a vector including a nucleic acid molecule which encodes a polypeptide including an amino acid sequence represented by the following formula (from N-terminus to C-terminus), or a cell transformed with the vector:

X4-X1-L-Q-C-V-K-N-P-E-X2-X3-S-C (formula II),

wherein
X4 is R or K;
X1 is F or L;
X2 is H or D; and
X3 is P or S.

According to a specific exemplary embodiment of the present disclosure, the vector or cell may include a nucleic acid molecule which encodes a polypeptide including an amino acid sequence selected from a group consisting of SEQ **ID** NOS: 4 to 6.

According to a specific exemplary embodiment of the present disclosure, the vector or cell may include a nucleic acid molecule which encodes a polypeptide including an amino acid sequence selected from a group consisting of SEQ **ID** NOS: 7 to 10.

According to a specific exemplary embodiment of the present disclosure, the vector or cell may include a nucleic acid molecule which encodes a polypeptide including an amino acid sequence selected from a group consisting of SEQ **ID** NOS: 11 to 15.

According to a specific exemplary embodiment of the present disclosure, the vector or cell may include a nucleic acid molecule which encodes a polypeptide including an amino acid sequence selected from a group consisting of SEQ **ID** NOS: 16 to 20.

According to a specific exemplary embodiment of the present disclosure, the vector or cell may include a nucleic acid molecule which encodes a polypeptide including an amino acid sequence selected from a group consisting of SEQ **ID** NOS: 21 to 25.

According to a specific exemplary embodiment of the present disclosure, the vector or cell may include a nucleic acid molecule which encodes a polypeptide including an amino acid sequence selected from a group consisting of SEQ **ID** NOS: 26 to 30.

According to a specific exemplary embodiment of the present disclosure, the vector or cell may include a nucleic acid molecule encoding a sequence of full-length CD38 (e.g., SEQ ID NOS: 31 to 36) or a fragment sequence thereof.

Examples of viruses that can be used as the vector in the present disclosure include, but are not limited to, retrovirus, herpes simplex virus, lentivirus, poxvirus, vaccinia virus, rhabdovirus, adenovirus, helper-dependent adenovirus, adeno-associated virus (AAV), baculovirus, and combinations thereof. In some aspects, the vector that may be used in the present disclosure includes a non-viral vector. Non-limiting examples of the vector include a plasmid, a cosmid, a yeast artificial chromosome (YAC), a bacteriophage, and combinations thereof.

In some aspects, the vector may further include a promoter sequence, an enhancer sequence, an exon sequence, an intron sequence, a signal sequence coding sequence, a splicing donor sequence, etc.

In some aspects, the cell described herein is capable of producing a protein encoded by the nucleic acid molecule in vitro. In certain aspects, the cell described herein is capable of producing the encoded protein in vivo. In some aspects, the cell described herein is capable of producing the encoded protein both in vitro and in vivo.

In some aspects, the cell that can be used to produce a protein encoded by the nucleic acid molecule (e.g., in vitro) includes a host cell. The term "host cell" as used herein is intended to include cells of any organism that are capable of being transduced with an expression construct (e.g., an AAV vector) to replicate the expression construct or express a gene encoded by the expression construct. The cells include eukaryotic cells and prokaryotic cells. The term "transduction" as used herein is intended to include transfection and transformation. The host cell can be transduced, transfected or transformed by the expression construct described above. This process involves the transfer or introduction of exogenous nucleic acid molecules into a host cell. In some aspects, the host cell is an isolated host cell. In some aspects, the host cell is a transformed isolated host cell.

In some aspects, the host cell is a eukaryotic cell. In some aspects, the host cell is selected from a group consisting of a mammalian cell, an insect cell, a yeast cell, a transgenic mammalian cell, and a plant cell. In some aspects, the host cell is a prokaryotic cell. In some aspects, the prokaryotic cell is a bacterial cell.

In some aspects, the host cell is an insect cell. In some aspects, the insect cell is Sf9. In some aspects, the host cell is a mammalian cell. Non-limiting examples of the mammalian cell that can be used in the present disclosure include HEK293, HeLa, ARPE-19, RPE-1, HepG2, Hep3B, Huh-7, C8D1a, Neuro2A, CHO, MES13, BHK-21, COS7, COP5, A549, MCF-7, HC70, HCC1428, BT-549, PC3, LNCaP, Capan-1, Panc-1, MIA PaCa-2, SW480, HCT166, LoVo, A172, MKN-45, MKN-74, Kato-III, NCI-N87, HT-144, SK-MEL-2, SH-SY5Y, C6, HT-22, PC-12 and NIH3T3 cells, and combinations thereof.

In some aspects, the cell that can be used to produce a protein encoded by a foreign gene described herein (e.g., in vivo) includes a human cell. In some aspects, the human cell is a cell of a subject to which the nucleic acid molecule described herein has been administered. In certain aspects, the human cell is derived from a donor (e.g., a healthy human subject).

### V. Pharmaceutical composition

The present disclosure relates to a pharmaceutical composition for preventing or treating a disease or a disorder, which contains a polypeptide including an amino acid sequence of CD38 (e.g., soluble CD38) or a fragment (functional fragment) thereof, a nucleic acid molecule encoding the polypeptide, a vector including the nucleic acid molecule, or a cell transformed with or including the vector, and a pharmaceutically acceptable carrier.

In some aspects, the disease or disorder is an autoimmune disease.

Examples of the autoimmune disease include, but are not limited to, rheumatoid arthritis, autoimmune thyroiditis, psoriasis, Crohn's disease, ulcerative colitis, lupus (systemic lupus erythematosus), uveitis, dry eye, allergies, atopic dermatitis, rhinitis, asthma, multiple sclerosis, or type 1 diabetes.

In some aspects, the disease or disorder is an inflammatory bowel disease.

Examples of the inflammatory bowel disease include, but are not limited to, ulcerative colitis or Crohn's disease.

The pharmaceutically acceptable carrier that can be used in the present disclosure is one commonly used in formulations. Examples of the pharmaceutically acceptable carrier include, but are not limited to, lactose, dextrose, sucrose, sorbitol, mannitol, starch, gum acacia, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, mineral oil, etc. The pharmaceutical composition of the present disclosure may further contain one or more additive selected from a group consisting of a lubricant, a wetting agent, a sweetener, a flavoring agent, an emulsifier, a suspending agent, and a preservative. Details of suitable pharmaceutically acceptable carriers and formulations can be found in Remington's Pharmaceutical Sciences (19th ed., 1995).

The pharmaceutical composition of the present disclosure is formulated to be compatible with its intended route of administration. The route of administration includes oral or parenteral administration. Examples of suitable parenteral routes of administration include intravenous infusion, transdermal administration, subcutaneous infusion, intramuscular infusion, intraocular (e.g., any administration that can be delivered to sub-tenon, subconjunctival, suprachoroidal, suprachoroidal, subretinal, intravitreal and similar locations) infusion, eye drop administration, intracerebroventricular injection, intrathecal injection, intraamniotic injection, intraarterial injection, intraarticular injection, intracardiac injection, intracavernosal injection, intracerebral injection, cistern injection, intracoronary injection, intracranial injection, intrathecal injection, epidural injection, intrahippocampal injection, intranasal injection, intraosseous injection, intraperitoneal injection, intrathoracic injection, intraspinal injection, intrathoracic injection, intrathymic injection, intrauterine injection, intravaginal injection, intraventricular injection, intravesical injection, subconjunctival injection, intratumoral injection, topical injection, intraperitoneal injection, and combinations thereof.

In some aspects, the pharmaceutical composition is administered at a daily dosage of 0.0001 to 100 mg/kg.

The pharmaceutical composition of the present disclosure may be formulated together with one or more pharmaceutically acceptable carrier and/or excipient. The pharmaceutical composition may be provided in a unit dosage form or may be dispensed into a multi-dose container. The formulation may be in the form of a solution, suspension or emulsion in an oil or aqueous medium, an extract, a powder, a granule, a tablet, or a capsule. The formulation may further contain a dispersant or a stabilizer.

### VI. Use

The polypeptide including CD38 (e.g., soluble CD38) or a fragment thereof according to the present disclosure, a nucleic acid encoding the polypeptide, a vector including the nucleic acid, a cell including or transformed with the nucleic acid or vector, and the method described herein have many utilities in vitro and in vivo. For example, the polypeptide, polynucleotide, vector, and cell in vitro or ex vivo described herein can be administered, for example, in vivo, to prevent or treat a disease. Thus, in some aspects, the present disclosure provides a therapeutic use of any of the polypeptide or polynucleotide described herein, the vector described herein, the cell described herein, and the pharmaceutical composition described herein. In some aspects, the present disclosure provides a method for preventing or treating an autoimmune disease in a subject in need thereof, which includes administering to the subject the polypeptide, polynucleotide, vector, cell, or pharmaceutical composition disclosed herein.

In some aspects, there may be provided a method for preventing or treating a disease or a disorder, which includes administering the polypeptide, a nucleic acid molecule encoding the polypeptide, a vector including the nucleic acid molecule, or an isolated cell including the vector to a subject in need thereof.

In some aspects, there may be provided a method for preventing or treating an autoimmune disease or an inflammatory bowel disease, which includes administering i) the polypeptide, a nucleic acid molecule encoding the polypeptide, a vector including the nucleic acid molecule, or an isolated cell including the vector, or ii) a pharmaceutical composition containing the polypeptide, the nucleic acid molecule, the vector, or the isolated cell to a subject in need thereof.

In some aspects, there may be provided a use of i) the polypeptide, the nucleic acid molecule encoding the polypeptide, the vector including the nucleic acid molecule, or the isolated cell including the vector, or ii) a pharmaceutical composition containing the polypeptide, the nucleic acid molecule, the vector, or the isolated cell for the prevention or treatment of an autoimmune disease or an inflammatory bowel disease.

The disease or disorder is not limited, but is specifically an autoimmune disease, more specifically one selected from a group consisting of rheumatoid arthritis, autoimmune thyroiditis, psoriasis, Crohn's disease, ulcerative colitis, lupus (systemic lupus erythematosus), uveitis, dry eye, allergies, atopic dermatitis, rhinitis, asthma, multiple sclerosis, or type 1 diabetes.

Additionally, the disease or disorder may specifically be an inflammatory bowel disease, more specifically ulcerative colitis or Crohn's disease.

In some aspects, the method may further include administering an additional therapeutic agent (e.g., an immunosuppressant) to the subject. In some aspects, the additional therapeutic agent can be administered to the subject concurrently with, prior to, or after administration of the polypeptide, polynucleotide, vector, cell, recombinant virus, or pharmaceutical composition.

The disease that can be prevented, improved or treated by the present disclosure is not limited but include all diseases that require a reduction in the number of drug administrations.

In some aspects, the polypeptide includes an amino acid sequence represented by the following formula (from N-terminus to C-terminus):

X1-L-Q-C-V-K-N-P-E-X2-X3-S-C (formula I),

wherein
X1 is F or L;
X2 is H or D; and
X3 is P or S.

In some aspects, the polypeptide may include an amino acid sequence selected from a group consisting of SEQ ID NOS: 1 to 3.

In some aspects, the polypeptide includes an amino acid sequence represented by the following formula (from N-terminus to C-terminus):

X4-X1-L-Q-C-V-K-N-P-E-X2-X3-S-C (formula II),

wherein
X4 is R or K;
X1 is F or L;
X2 is H or D; and
X3 is P or S.

In some aspects, the polypeptide may include an amino acid sequence selected from a group consisting of SEQ ID NOS: 4 to 30.

In some aspects, the polypeptide may consist of 13 to 300, 13 to 250, 13 to 240, 13 to 230, 13 to 220, 13 to 210, 13 to 200, 13 to 190, 13 to 180, 13 to 170, 13 to 160, 13 to 150, 13 to 140, 13 to 130, 13 to 120, 13 to 110, 13 to 100, 13 to 90, 13 to 80, 13 to 70, 13 to 60, 13 to It may consist of 50, 13 to 49, 13 to 48, 13 to 47, 13 to 46, 13 to 45, 13 to 44, 13 to 43, 13 to 42, 13 to 41, 13 to 40, or 13 to 39 amino acids.

In some aspects, the polypeptide is a fragment of CD38.

In some aspects, the CD38 is soluble CD38.

In some aspects, the CD38 may be derived from dog, horse, mouse, rat, human or chimpanzee.

### VII. Preparation method

The present disclosure relates to a method for preparing a pharmaceutical composition for preventing or treating a disease or a disorder, which includes a step of preparing a polypeptide including an amino acid sequence of CD38 (e.g., soluble CD38) or a fragment (functional fragment) thereof, a nucleic acid molecule encoding the polypeptide, a vector including the nucleic acid molecule, or a cell transformed with or including the vector.

### [Advantageous Effects]

The features and advantages of the present disclosure may be summarized as follows:
(i) The present disclosure provides a polypeptide including a fragment of CD38.
(ii) The present disclosure also provides a composition for preventing or treating an autoimmune disease, which contains CD38 or a fragment thereof.
(iii) According to the present disclosure, various diseases related to autoimmunity can be treated using a polypeptide including soluble CD38 or a fragment thereof, or a nucleic acid molecule encoding the same.

### [Brief Description of Drawings]

FIG. 1 illustrates a first functional domain screening test using CD38 recombinant protein.
FIG. 2 illustrates a second sCD38 functional domain screening test following the first sCD38 functional domain screening.
FIG. 3 illustrates a third sCD38 functional domain screening test following the second sCD38 functional domain screening.
FIG. 4 shows a result of testing the efficacy of the sCD38 functional domain in an animal model of autoimmune uveitis.
FIG. 5 shows a result of comparing the amount of Treg cells in an autoimmune uveitis animal model of the sCD38 functional domain.
FIG. 6 shows a result of comparing cytokine levels in an autoimmune uveitis animal model of the sCD38 functional domain.
FIG. 7 shows a result of testing the efficacy of the sCD38 functional domain in an animal model of rheumatoid arthritis.
FIG. 8 shows a result of testing the efficacy of the sCD38 functional domain in an animal model of ulcerative colitis.
FIG. 9 shows a result of testing the efficacy of the human sCD38 functional domain in human dendritic cells.
FIG. 10 shows the results of comparing the homology of the interspecies sequences of CD38 of the present disclosure through alignment (human (59%), rat (88%), dog (57%), horse (58%), chimpanzee (58%) with respect to mouse).

### [Best Mode]

Hereafter, the present disclosure will be described in more detail through examples. These examples are intended solely to explain the present disclosure in more detail, and it will be obvious to those having ordinary knowledge in the art that the scope of the present disclosure is not limited by the examples.

### Examples

### Example 1. Isolation of mouse bone marrow cells and differentiation of DCs

To isolate bone marrow cells, mice (Damool Science, Korea) were anesthetized and sacrificed, and the femur and tibia were removed aseptically and the tissue attached to the bones was removed. After cutting off both ends of the bones, bone marrow was collected by slowly injecting 5-10 mL of a cell culture medium or a solution used for bone marrow separation into the medullary cavity at one end using a syringe needle, which was then filtered through a 70 µm cell strainer (SPL Life Sciences, Korea) and centrifuged to collect bone marrow cells. After treating with 1 mL of an RBC lysis solution (Merck, Germany) for 2-5 minutes to remove red blood cells, 10 mL of Dulbecco's phosphate-buffered saline (DPBS) (GIBCO, USA) was added, and bone marrow cells were collected through centrifugation. After washing the bone marrow cells 3 times with DPBS, 10 mL of a BMDC culture medium (RPMI-1640 (Welgene, Korea) containing 10% FBS (GIBCO, USA) and 10 ng/mL GM-CSF (Peprotech, USA)) was added to disperse the bone marrow cells into single cells. 1×10⁵ bone marrow cells were dispensed into a 24-well culture dish and differentiated in a 37 °C incubator maintained at 5% CO₂.

### Example 2. First functional domain screening test using CD38 recombinant protein

To screen the functional domain of sCD38 that induces dendritic cell tolerance, the domain of sCD38 was largely divided into three parts (CD38 amino acid sequence; 42-140 (1), 130-220 (2), 210-304 (3)). Using the three parts of sCD38, TNF-α secretion was observed as one of the indicators of the degree of DC tolerance induction.

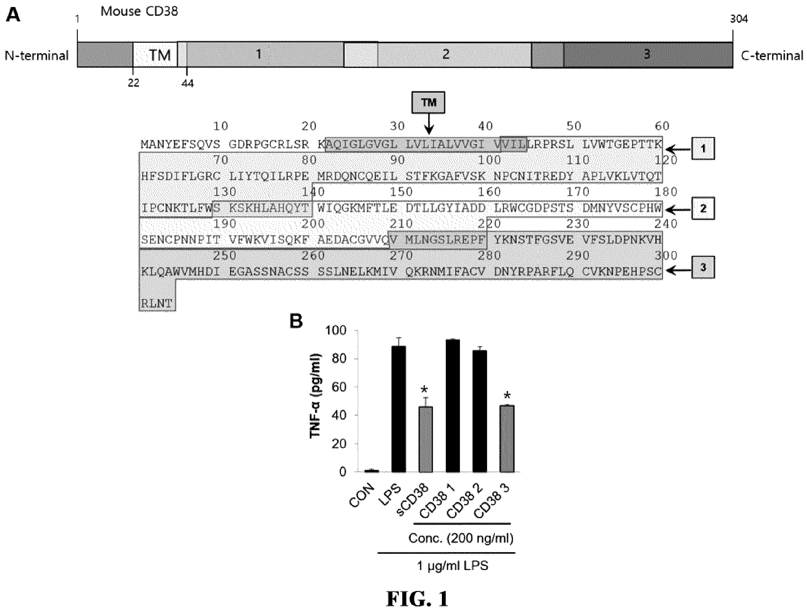

As shown in FIG. 1, the domain of sCD38 was divided into three parts and the ability of DCs to secrete TNF-α induced by LPS was observed. An *E*. *coli* expression system was used to express the sCD38 domain protein. The sCD38 domain protein expressed in *E*. *coli* was purified using a purification system and used for screening. For fast and easy screening of the domain protein of sCD38, an ELISA method that measures the pro-inflammatory cytokine TNF-α was adopted. When the DCs obtained by treating the bone marrow cells with GM-CSF are treated with LPS, the DCs differentiate into mature DCs and the secretion of TNF-α is increased. It was confirmed that TNF-α secretion from DCs induced by LPS was reduced by the treatment with sCD38 (SEQ ID NO: 33). As a result of treating with sCD38 functional domain, (1) 42-140, (2) 130-220, and (3) 210-304, it was confirmed that the TNF-α secretion from dendritic cells induced by LPS (Merck, Germany) was reduced by the sCD38 functional domain (3).

### Example 3. Second functional domain screening test after first functional domain screening

After the first screening of the sCE38 functional domain that induces dendritic cell tolerance, the selected domain was divided into two parts (C-term 226-265 and C-term 266-304 of CD38 210-304 (3), (SEQ ID NO: 26)). After the domain was divided into two parts, TNF-α secretion was observed as one of the indicators of the degree of DC tolerance induction.

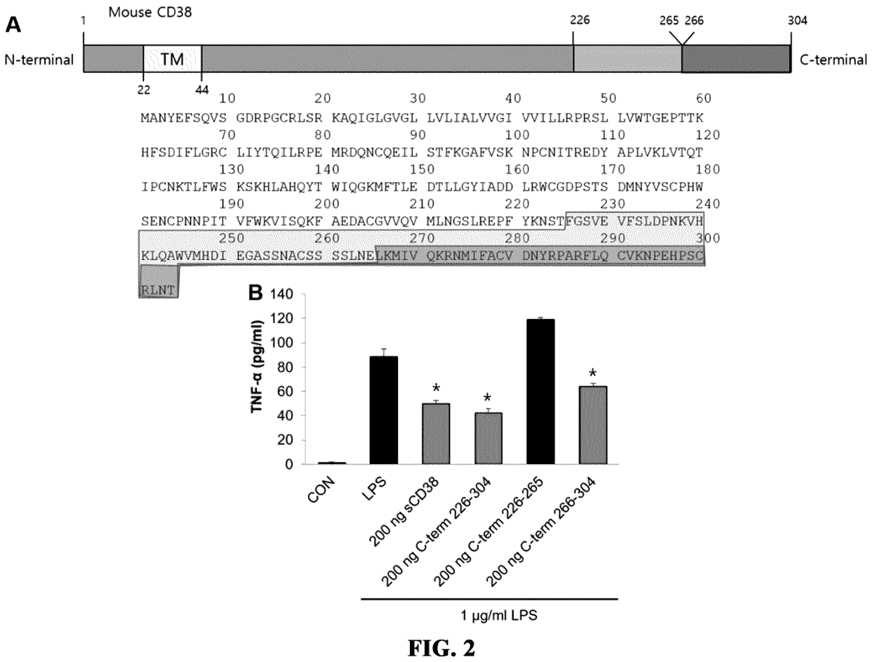

As shown in FIG. 2, the functional domain (3) of sCD38 confirmed in FIG. 1 was divided into two parts again, and the ability of DCs to secrete TNF-α by LPS was observed. An *E*. *coli* expression system was used to express the divided two parts of sCD38. As a result of treating with the divided two parts (C-term 226-265, C-term 266-304) of the sCD38 functional domain (3), it was confirmed that the TNF-α secretion from dendritic cells induced by LPS was reduced by the sCD38 functional domain C-term 266-304.

### Example 4. Third functional domain screening test after second functional domain screening

After the second screening of the sCE38 functional domains that induce dendritic cell tolerance, the screened domain was divided into seven parts and peptide synthesis was performed (peptide 1: 233-247 of sCD38 226-304, peptide 2: 251-265, peptide 3: 269-283, peptide 4: 266-304 (SEQ ID NO: 26), peptide 5 (M19): 286-304 (SEQ ID NO: 21), peptide 6 (M16): 287-302 (SEQ ID NO: 11), peptide 7 (M14): 287-300 (SEQ ID NO: 4)). Using the seven divided domain peptides, TNF-α secretion was observed as one of the indicators of the degree of DC tolerance induction. In addition, after synthesizing the domain peptide H18 (283-300) (SEQ ID NO: 18) of human CD38 at the same position as M19 (286-304) and treating dendritic cells with the same, the secretion of TNF-α was observed as one of the indicators of the degree of DC tolerance induction.

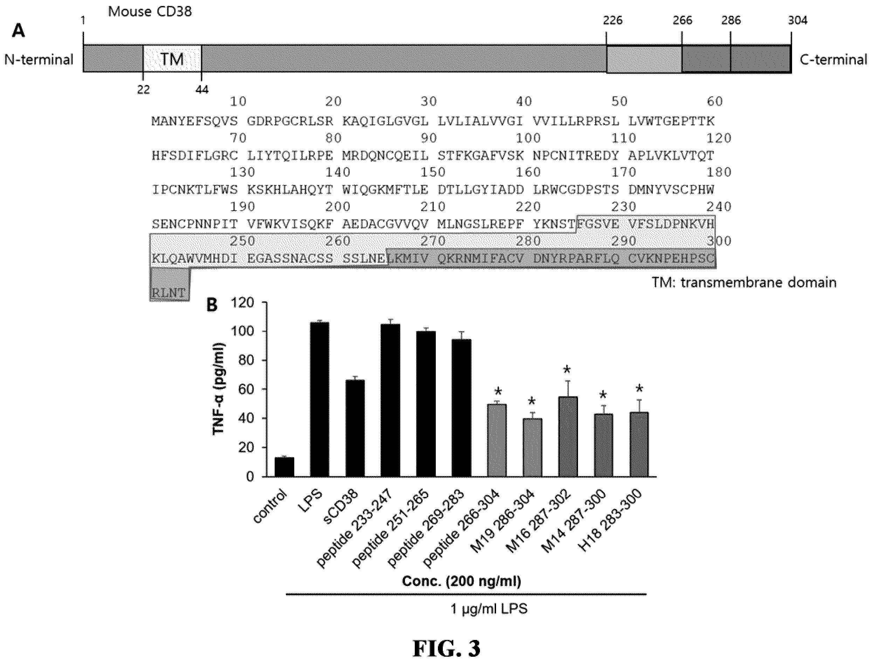

As shown in FIG. 3, the functional domain C-term 226-304 of sCD38 was produced again into several peptide fragments, and the ability of DCs to secrete TNF-α induced by LPS was observed. It was confirmed that the TNF-α secretion from dendritic cells induced by LPS was reduced by the sCD38 functional domain peptide when they were treated with short peptides (peptide 4 (266-304), M19 (286-304), M16 (287-302), M14 (287-300), H18 (283-300)).

### Example 5. Efficacy test of sCD38 functional domain in an animal model of autoimmune uveitis

Autoimmune uveitis was induced by intraperitoneal injection of interphotoreceptor retinoid-binding protein (IRBP) (Peptron, Korea) into the hind limb muscles of C57BL/6 mice (Damool Science, Korea) and intraperitoneal injection of pertussis toxin (PTX) (List Lab, USA). After 10 to 14 days, the presence or absence of autoimmune uveitis was confirmed in the eyes of the mice. The functional domain of sCD38 (226-304) was injected into the eye of the animal model that developed autoimmune uveitis twice a week to determine the progression of autoimmune uveitis. An autoimmune uveitis animal model to which the functional domain of sCD38 (226-304) was not injected was used as a control group, and animals to which the functional domain of sCD38 (226-304) was injected were used as a test group. The severity of autoimmune uveitis was measured for the experimental animals.

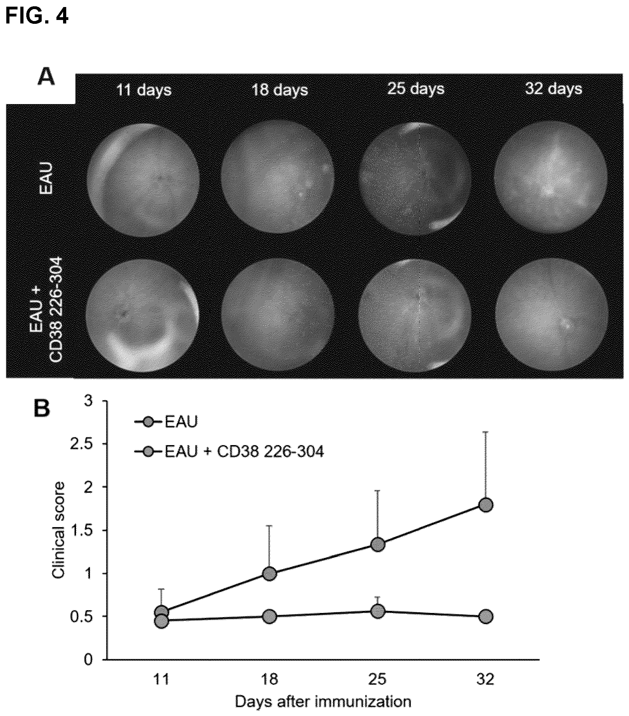

As shown in FIG. 4, when the eyes of the control animal model that developed autoimmune uveitis were compared with the eyes of the experimental animal model treated with the functional domain (226-304) of sCD38, it was confirmed that the progression of autoimmune uveitis was reduced in the eyes of the experimental animal model treated with the functional domain (226-304) of sCD38.

### Example 6. Comparison of amount of Treg cells in animal model of autoimmune uveitis depending on treatment with sCD38 functional domain

Next, to analyze the amount of Tregs in the experimental animals, after anesthetizing and sacrificing the experimental animals, the lymph nodes and eyeballs were isolated aseptically. The isolated lymph nodes or eyeballs were cut into small pieces using surgical scissors, placed in a 70 µm cell strainer (SPL Life Sciences, Korea), filtered into 10 mL of a cell culture medium (RPMI-1640 culture medium), and then centrifuged to collect lymph node cells or eye cells. To remove red blood cells contained in small amounts in the lymph node cells or eye cells, 1 mL of an RBC lysis solution was treated for 2-5 minutes. Then, after adding 10 mL of Dulbecco's phosphate-buffered saline (DPBS) (GIBCO, USA), the lymph node cells or eye cells were collected by centrifugation. After washing three times with DPBS, 10 mL of an RPMI culture medium (culture medium containing 10% FBS (GIBCO, USA)) was added to disperse the lymph node cells or eye cells into single cells. The lymph node cells or eye cells were divided into 2 to 5x10⁵ cells and the amount of Tregs in the lymph node cells or eye cells was analyzed by flow cytometry analysis.

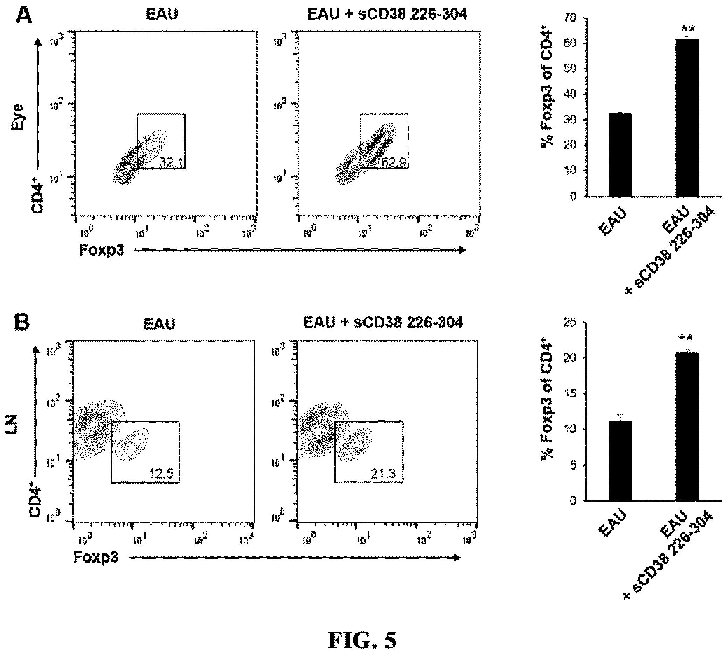

As shown in FIG. 5, as a result of comparing the eyes of the control animal model in which uveitis was induced with the eyes of the experimental animal model treated with the functional domain (226-304) of sCD38, it was confirmed that the amount of Treg cells increased in the eyes of the experimental animal model treated with the functional domain (226-304) of sCD38.

### Example 7. Comparison of cytokine level in animal model of autoimmune uveitis depending on treatment with sCD38 functional domain

Next, to analyze the amount of cytokines (IL-17, INF-γ, etc.) secreted by the experimental animals, after anesthetizing and sacrificing the experimental animals, splenocytes were isolated aseptically. The isolated splenocytes were cut into small pieces using surgical scissors, placed in a 70 µm cell strainer (SPL Life Sciences, Korea), filtered into 10 mL of a cell culture medium (RPMI-1640 culture medium), and then centrifuged to collect the splenocytes. To remove red blood cells contained in the splenocytes, 1 mL of an RBC lysis solution was treated for 2-5 minutes. Then, after adding 10 mL of Dulbecco's phosphate-buffered saline (DPBS) (GIBCO, USA), the splenocytes were collected by centrifugation. After washing three times with DPBS, 10 mL of an RPMI culture medium (culture medium containing 10% FBS (GIBCO, USA)) was added to disperse the splenocytes into single cells. The splenocytes were divided into 1x10⁶ cells and the amount of cytokines was analyzed.

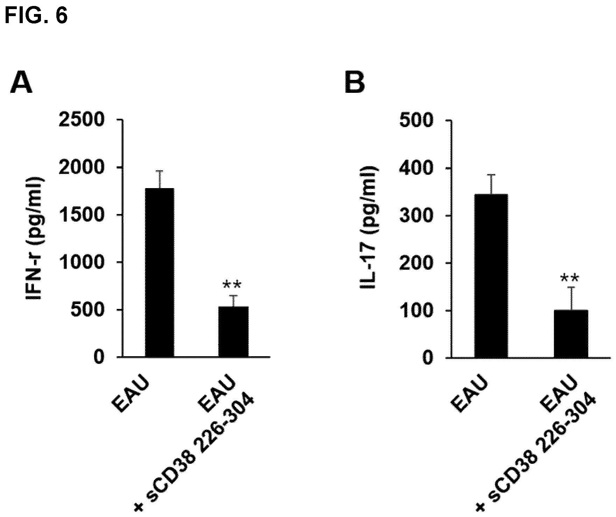

As shown in FIG. 6, as a result of comparing the eyes of the control animal model in which uveitis was induced with the eyes of the experimental animal model treated with the functional domain (226-304) of sCD38, it was confirmed that the amount of cytokines was reduced in the eyes of the experimental animal model treated with the functional domain (226-304) of sCD38.

### Example 8. Efficacy of sCD38 functional domain in animal model of rheumatoid arthritis

For induction of collagen-induced arthritis (CIA), 8-9 week-old male DBA/1 mice (Central Lab. Animal, Korea) were immunized with bovine type II collagen (CII) (Central Lab. Animal, Korea). CII was dissolved in 0.05 N acetic acid at a concentration of 2 mg/mL and emulsified at a ratio of 1:1 with complete Freund's adjuvant (CFA) (Central Lab. Animal, Korea) at 4 °C. For the first immunization, 150 µL of a mixture of 100 µg CII and 100 µg CFA was injected intradermally into the tail of the mice. Three weeks later, before booster injection, the sCD38 functional domain (sCD38-3) (226-304) or saline was injected into the ankle joint of the mice, and an equal volume of CII, emulsified at a ratio of 1:1 with incomplete Freund's adjuvant (IFA) (Merck, Germany), was injected into the tail (booster injection). The sCD38 functional domain (sCD38-3) was injected three more times, once a week. After 4 weeks of the booster injection, the mice were euthanized for analysis. After the booster injection, the thickness of the hind ankle of the animal model was measured using a vernier caliper.

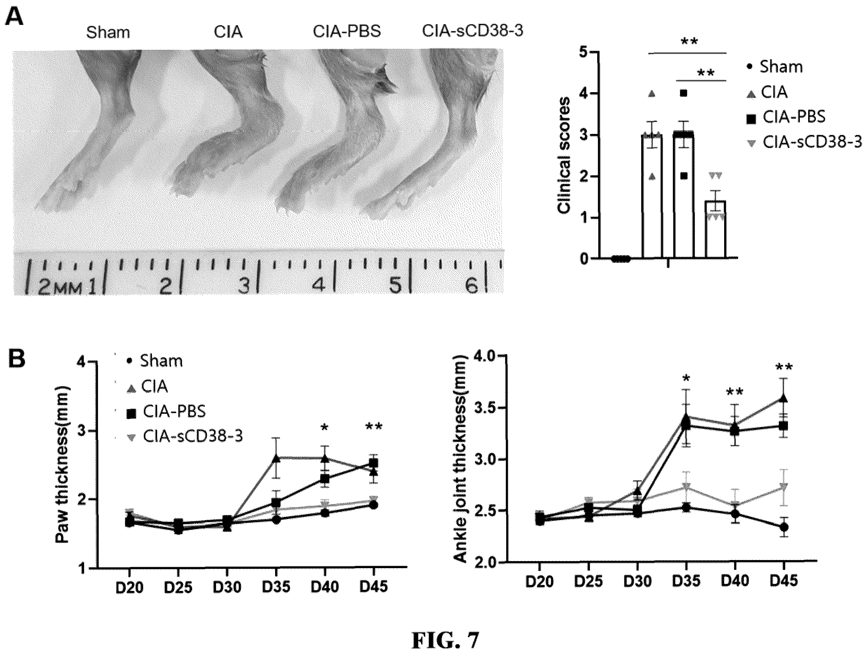

As shown in FIG. 7, when the thickness of the hind ankle of the control animal model with rheumatoid arthritis was compared with the thickness of the hind ankle of the experimental animal model treated with the functional domain of sCD38 (sCD38-3), it was confirmed that the thickness of the hind ankle of the experimental animal model treated with the functional domain of sCD38 (sCD38-3) was significantly reduced compared to the thickness of the hind ankle of the animal model with rheumatoid arthritis.

### Example 9. Efficacy of sCD38 functional domain in animal model of ulcerative colitis

A control ulcerative colitis animal model was established by allowing C57BL/6 mice (Damool Science, Korea) to drink drinking water containing 2-3% dextran sulfate sodium (DSS) (MP Biomedicals, China) for 6-7 days. Administration of DSS increases the level of inflammatory cytokines and causes colitis in mice. The experimental animal model was treated with DSS for 6-7 days, and 20 µg of the sCD38 functional domain (M19 peptide (Peptron, Korea)) was injected intrarectally daily for 2 weeks. Then, it was determined whether the progression of ulcerative colitis was reduced. LiCl (Merck, Germany) was used instead of the sCD38 functional domain (M19 peptide) for a positive control group.

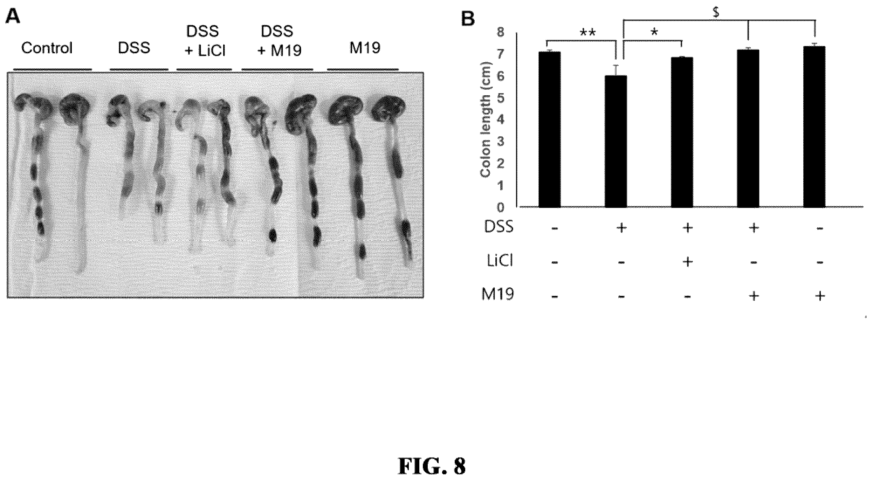

As shown in FIG. 8, it was confirmed that the length of the colon of the control animal model that developed ulcerative colitis was shorter than the length of the colon of the animal that did not develop ulcerative colitis. In contrast, it was confirmed that the colon length of the experimental animal model treated with the sCD38 functional domain (M19 peptide) was not shortened.

### Example 10. Isolation of monocytes from human whole blood and differentiation of monocyte-derived dendritic cells

To isolate human monocytes, 20 mL of whole blood was withdrawn and mix with an equal volume of PBS. After adding 15 mL of Ficoll (GE HealthCare, USA) to a 50-mL tube, 15 mL of diluted whole blood was slowly added onto the Ficoll. Likewise, an equal amount of the remaining whole blood was dispensed slowly into a 50-mL tube containing 15 mL of Ficoll. Centrifugation was performed at 1000 x g for 20 minutes in a centrifuge at room temperature. After carefully removing plasma from the isolated whole blood, a portion including peripheral blood mononuclear cells (PBMCs) was transferred to a new 50-mL tube. After adding 10 mL of PBS to the tube containing PBMCs and mixing gently, centrifugation was performed at 400 x g for 10 minutes at room temperature. After carefully removing the supernatant, 1 mL of an RPMI 1640 medium containing 10% FBS was added to the PBMC pellets, and the cells were counted. After adding an RPMI-1640 medium (Welgene, Korea) containing 10% FBS to 1x10⁶ cells per mL, 100 ng/mL GM-CSF (Peprotech, USA) and 50 ng/mL IL-4 (Peprotech, USA) were mixed. After adding 500 µL of PMBC to each well of a 24-well plate, the cells were differentiated in an incubator at 37 °C maintained at 5% CO₂ for 5 days.

### Example 11. Efficacy of sCD38 functional domain in human dendritic cells

It was confirmed whether the sCD38 functional domain that induces tolerance of dendritic cells is also effective in human dendritic cells. To identify the functional domain of human sCD38, the C-terminal portion of human CD38 (H18 (283-300), H13 (284-296) (SEQ ID NO: 2)) were prepared into peptides. Human sCD38 and M19 peptides were treated as positive controls, and TNF-α secretion was observed using H18 and H13 peptides as one of the indicators of the degree of DC tolerance induction.

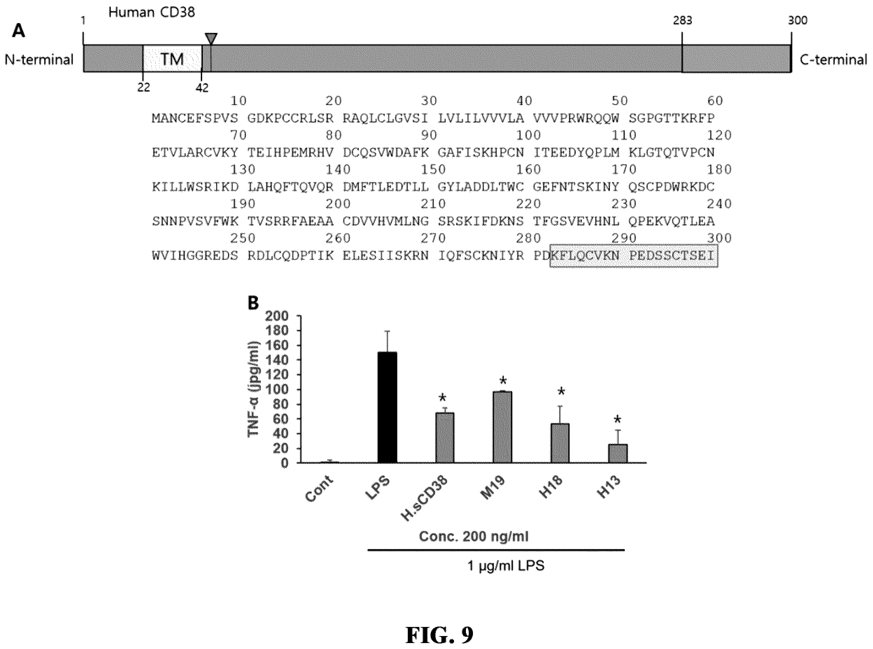

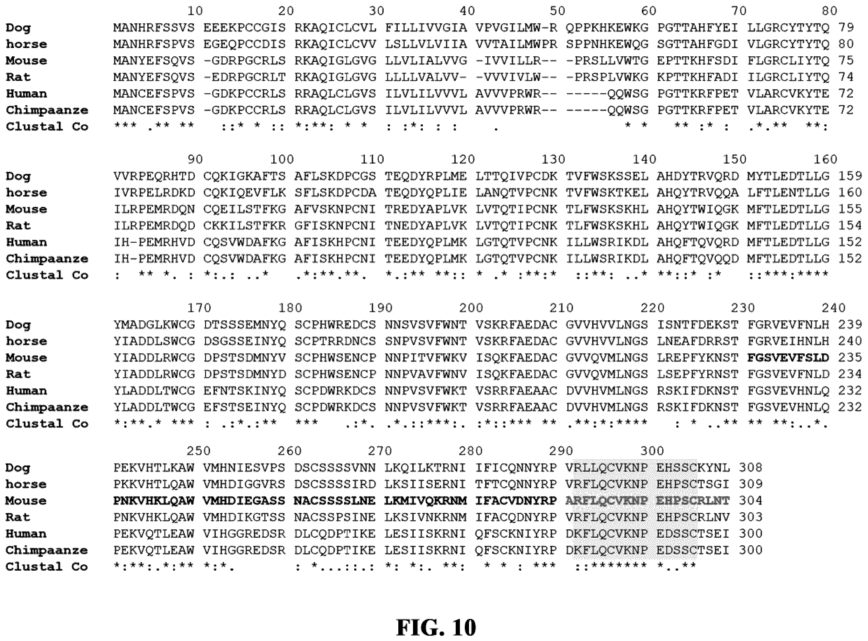

As shown in FIGS. 9 and 10, H18 (283-300) and H13 (284-296) at the C-term of human sCD38, which are located at the same position as the C-term of mouse sCD38, were prepared into peptides, and the ability of human dendritic cells to secrete TNF-α induced by LPS was observed. It was confirmed that the TNF-α secretion induced by LPS from human dendritic cells was reduced by the sCD38 functional domain peptides when the human sCD38 and M19 peptides were treated as positive controls and they were treated with the H18 and H13 peptides.

Hereinafter, formulation examples of a pharmaceutical composition containing the composition of the present disclosure will be described. However, they are not intended to limit the present disclosure.

### Formulation Example 1. Preparation of powder

| | |
|---|---|
| NAD glycohydrolase (NADase) inhibitor | 20 mg |
| Lactose | 100 mg |
| Talc | 10 mg |

The above ingredients were mixed and filled into a sealed bag to prepare a powder.

### Formulation Example 2. Preparation of tablet

| | |
|---|---|
| NAD glycohydrolase (NADase) inhibitor | 10 mg |
| Corn starch | 100 mg |
| Lactose | 100 mg |
| Magnesium stearate | 2 mg |

After mixing the above ingredients, a tablet was prepared by compressing the mixture according to a common method for preparing tablets.

### Formulation Example 3. Preparation of capsule

| | |
|---|---|
| NAD glycohydrolase (NADase) inhibitor | 10 mg |
| Crystalline cellulose | 3 mg |
| Lactose | 14.8 mg |
| Magnesium stearate | 0.2 mg |

The above ingredients were mixed according to the conventional capsule preparation method and filled into a gelatin capsule to prepare a capsule.

### Formulation Example 4. Preparation of injection

| | |
|---|---|
| NAD glycohydrolase (NADase) inhibitor | 10 mg |
| Mannitol | 180 mg |
| Sterile distilled water for injection | 2974 mg |
| Na₂HPO₄·2H₂O | 26 mg |

According to a common injection preparation method, the above ingredients were prepared into an injection per ampoule (2 mL).

### Formulation Example 5. Preparation of liquid preparation

| | |
|---|---|
| NAD glycohydrolase (NADase) inhibitor | 20 mg |
| High-fructose corn syrup | 10 g |
| Mannitol | 5 g |
| Purified water | Adequate |

While the exemplary embodiments of the present disclosure have been described, those skilled in the art will be able to modify and change the present disclosure in various ways by adding, changing, deleting or adding components, etc., within the scope that does not depart from the spirit of the present disclosure described in the claims, and this will also be considered to be included within the scope of the rights of the present disclosure.

## Claims

1. A polypeptide comprising an amino acid sequence represented by the following formula (from N-terminus to C-terminus):
X1-L-Q-C-V-K-N-P-E-X2-X3-S-C (formula I),
wherein
X1 is F or L;
X2 is H or D; and
X3 is P or S.

2. The polypeptide according to claim 1, wherein the polypeptide comprises an amino acid sequence selected from a group consisting of SEQ ID NOS: 1 to 3.

3. The polypeptide according to claim 1, wherein the polypeptide comprises an amino acid sequence represented by the following formula (from the N-terminus to the C-terminus):
X4-X1-L-Q-C-V-K-N-P-E-X2-X3-S-C (formula II),
wherein
X4 is R or K;
X1 is F or L;
X2 is H or D; and
X3 is P or S.

4. The polypeptide according to claim 3, wherein the polypeptide comprises an amino acid sequence selected from a group consisting of SEQ ID NOS: 4 to 30.

5. The polypeptide according to claim 1, wherein the polypeptide consists of 13 to 300, 13 to 250, 13 to 240, 13 to 230, 13 to 220, 13 to 210, 13 to 200, 13 to 190, 13 to 180, 13 to 170, 13 to 160, 13 to 150, 13 to 140, 13 to 130, 13 to 120, 13 to 110, 13 to 100, 13 to 90, 13 to 80, 13 to 70, 13 to 60, 13 to 50, 13 to 49, 13 to 48, 13 to 47, 13 to 46, 13 to 45, 13 to 44, 13 to 43, 13 to 42, 13 to 41, 13 to 40, or 13 to 39 amino acids.

6. The polypeptide according to claim 1, wherein the polypeptide is a fragment of CD38.

7. The polypeptide according to claim 6, wherein the CD38 is soluble CD38.

8. The polypeptide according to claim 6, wherein the CD38 is derived from dog, horse, mouse, rat, human or chimpanzee.

9. A nucleic acid molecule encoding the polypeptide according to claim 1.

10. A vector comprising the nucleic acid molecule according to claim 9.

11. A host cell comprising the vector according to claim 10.

12. A host cell transformed with the vector according to claim 10.

13. A pharmaceutical composition for preventing or treating an autoimmune disease or an inflammatory bowel disease, comprising the polypeptide according to claim 1, a nucleic acid molecule encoding the polypeptide, a vector comprising the nucleic acid molecule, or an isolated cell comprising the vector, and a pharmaceutically acceptable carrier.

14. A method for preventing or treating an autoimmune disease or an inflammatory bowel disease, comprising a step of administering i) the polypeptide according to claim 1, a nucleic acid molecule encoding the polypeptide, a vector comprising the nucleic acid molecule, or an isolated cell comprising the vector, or ii) a pharmaceutical composition comprising the polypeptide, the nucleic acid molecule, the vector, or the isolated cell to a subject in need thereof.

15. A use of i) the polypeptide according to claim 1, a nucleic acid molecule encoding the polypeptide, a vector comprising the nucleic acid molecule, or an isolated cell comprising the vector, or ii) a pharmaceutical composition comprising the polypeptide, the nucleic acid molecule, the vector, or the isolated cell for prevention or treatment of an autoimmune disease or an inflammatory bowel disease.
